(19) Europäisches Patentamt
European Patent Office
Office européen des brevets

(11) EP 4 153 320 B1

(12) **EUROPEAN PATENT SPECIFICATION**

(45) Date of publication and mention of the grant of the patent:
**08.07.2026 Bulletin 2026/28**

(21) Application number: **21719169.1**

(22) Date of filing: **21.04.2021**

(51) International Patent Classification (IPC):
***A61Q 11/00*** *(2006.01)* ***A61K 8/73*** *(2006.01)*
***A61K 8/25*** *(2006.01)*

(52) Cooperative Patent Classification (CPC):
**A61K 8/735; A61K 8/25; A61Q 11/00**

(86) International application number:
**PCT/EP2021/060310**

(87) International publication number:
**WO 2021/233630 (25.11.2021 Gazette 2021/47)**

(54) **ORAL CARE COMPOSITION**
MUNDPFLEGEZUSAMMENSETZUNG
COMPOSITION D'HYGIÈNE BUCCALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.05.2020 PCT/CN2020/091561**
**06.07.2020 EP 20184183**

(43) Date of publication of application:
**29.03.2023 Bulletin 2023/13**

(73) Proprietors:
- **Unilever IP Holdings B.V.**
  **6708 WH Wageningen (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **LIU, Weining**
  **Shanghai, 200335 (CN)**
- **WANG, Weichong**
  **Shanghai, 200335 (CN)**
- **ZHOU, Huanjun**
  **Shanghai, 201803 (CN)**

(74) Representative: **Unilever Patent Group**
**Bronland 14**
**6708 WH Wageningen (NL)**

(56) References cited:
**EP-A1- 3 056 195**
**WO-A1-2017/198392**
**WO-A1-2018/156881**
**WO-A2-2008/005509**
**CN-A- 106 214 496**
**CN-A- 109 431 857**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical Field of the Invention

The present invention relates to oral care compositions such as toothpastes, gums, mouth-washes and the like. In particular, the present invention relates to an oral care composition for remineralization and/or hypersensitivity relates issues. The invention also relates to the use of such composition for remineralizing and/or reducing hypersensitivity of teeth of an individual.

### Background of the Invention

Tooth hypersensitivity is a temporary induced pain sensation. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dento-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treating tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

Hyaluronic acid (also called as hyaluronan) is a straight chain, glycosaminoglycan polymer of the extracellular matrix composed of repeating units of the disaccharide [-D-glucuronic acid-$\beta$1,3-N-acetyl-D-glucosamine-$\beta$1,4-]n, and is identified via CAS number 9004-61-9, and it can be found in all tissues and body fluids of vertebrates as well as in some bacteria. It is a linear polymer of exceptional molecular weight, especially abundant in loose connective tissue.

Hyaluronic acid is regarded as a most effective moisturizer even found in natural world, and thus is widely used in cosmetics, for example, as a super moisturizer. However, hyaluronic acid is seldom found in an oral care product.

WO2017/198392 discloses calcium silicate particles for use in the treatment of dentinal hypersensitivity.

US2019262254 discloses an oral care composition comprising hemp seed oil, caprylyl glycol and optionally hyaluronic acid and aloe vera for addressing oral dryness and discomfort. The amount of the hyaluronic acid is from 0.01 to 0.1% by weight of the composition.

US 2013/164359 discloses an oral care composition comprising a coated particle to produce calcium and phosphate in situ for long term whiteness. This composition may contain calcium silicate, as disclosed in the specification (Paragraph 0087).

None of the references above describes an oral care composition comprising calcium silicate and hyaluronic acid and/or its derivatives which is particularly suitable for enhancing the efficacy for tubule blockage and/or tooth remineralization.

The present inventors have now found unexpectedly that an oral care composition comprising calcium silicate and hyaluronic acid and/or its derivatives is more efficient for treating tooth hypersensitivity. The oral care composition exhibited excellent tubule blockage efficacy to reduce tooth hypersensitivity due to the enhanced tooth remineralization efficacy on tooth surface.

### Summary of the Invention

In a first aspect, the present invention is directed to an oral care composition comprising:

a) calcium silicate; and
b) hyaluronic acid and/or its derivatives;

wherein the molecular weight of the hyaluronic acid and/or its derivatives ranges from 500,000 Dalton to 3,000,000 Dalton,

as disclosed in the present claims.

In a second aspect, the present invention is directed to a packaged oral care product comprising the oral care composition of the first aspect of this invention.

In a third aspect, the present invention is directed to said composition for use in a method of remineralizing and/or reducing hypersensitivity of teeth of an individual comprising the step of applying the oral care composition of any embodiment of the first aspect to at least one surface of the teeth of the individual.

All other aspects of the present invention will more readily become apparent upon considering the detailed description and examples which follow.

## Terminology

Remineralization

"Remineralization", as used herein, means *in situ* (i.e. in the oral cavity) generation of calcium phosphate on teeth to reduce the likelihood of tooth hypersensitivity, tooth decay, regenerate enamel and/or improve the appearance of teeth by whitening through the generation of such new calcium phosphate.

Tooth hypersensitivity

Tooth hypersensitivity refers to a temporary induced pain sensation for tooth; for example, human tooth may be sensitive to factors such as temperature, pressure or chemical action.

Deposition Aid

Deposition aid, as used herein, means a material which aids deposition of actives such as calcium silicate onto the tooth surface during use of the composition.

Tubule blocking agent

Substances physically block the exposed ends of the dentinal tubules, thereby reduce dentinal fluid movement and reduce the irritation associated with the shear stress described by hydrodynamic theory.

Dual-Phase

"Dual-Phase" for the purposes of the present invention means a composition having two independent phases which are physically separate.

Dentifrice

"Dentifrice" for the purposes of the present invention means a paste, powder, liquid, gum or other preparation for cleaning the teeth or other surfaces in the oral cavity.

Toothpaste

"Toothpaste" for the purpose of the present invention means a paste or gel dentifrice for use with a toothbrush. Especially preferred are toothpastes suitable for cleaning teeth by brushing for about two minutes.

Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

Water of Hydration

"Water of hydration", as used herein, refers to water chemically combined with a substance in a way that it can be removed by heating without substantially changing the chemical composition of the substance. In particular, water which could only be removed when heated above 200°C. The water loss is measured using thermo gravimetric analysis (TGA)

with a Netzsch TG instrument. The TGA is conducted under an $N_2$ atmosphere with heating rate of 10 degree/min in the range of 30 to 900°C.

Viscosity

Viscosity of a toothpaste is the value taken at room temperature (25°C) with a Brookfield Viscometer, Spindle No.4 and at a speed of 5 rpm. Values are quoted in centipoises (cP=mPa.s) unless otherwise specified.

Molecular weight

"Molecular weight", as used herein, refers to the viscosity average molecular weight of a given polymer. The viscosity average molecular weight of hyaluronic acid and/or its derivatives herein is measured by using an Ubbelohde viscometer to obtain an intrinsic viscosity [η], and the molecular weight is calculated by the equation:

$$M_v = \left(\frac{[\eta]\times 10^5}{36}\right)^{\frac{1}{0.78}}$$

Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

Detailed Description

Now it has been found expectedly that the tooth remineralization efficacy can be enhanced by using calcium silicate in combination with hyaluronic acid and/or its derivatives. Besides, the present inventors have further found that such an oral care composition is effective in blocking dentinal tubules to reduce tooth hypersensitivity which is beneficial for treating tooth hypersensitivity.

CALCIUM SILICATE

The calcium silicate is of a basic formula $CaSiO_3$, and has very low water solubility or is substantially insoluble in water. The calcium silicate is present as a composite material of calcium oxide-silica ($CaO-SiO_2$), which is described, for example, in international patent application published as WO 2008/068248(Unilever), which is hereby incorporated by reference in its entirety.

For the calcium silicate composite material, the atom ratio of calcium to silicon (Ca:Si) may be from 1:10 to 3:1. The Ca:Si ratio is preferably 1:5 to 2:1, and more preferably, from 1:3 to 2:1, and most preferably, from about 1:2 to 2:1. In another preferred embodiment, the Ca:Si ratio may be from 1:30 to 3:1, preferably from 1:20 to 2:1 and more preferably from 1:10 to 1:1, and most preferably, from 1:7 to 1:1.5.The calcium silicate may comprise mono-calcium silicate, bi-calcium silicate, or tri-calcium silicate. The calcium silicate may be in a crystalline or amorphous state; furthermore, calcium silicate can even be in a mesoporous state as well.

Calcium silicate can be in the form of calcium silicate hydrate, or can be anhydrous calcium silicate or is not hydrated.

For the anhydrous calcium silicate, in addition to calcium oxide, silica, the calcium silicate is substantially free of other elements. The calcium silicate consists of (or at least consists essentially of) calcium oxide, silica.

In an embodiment of the invention, the calcium silicate for use in the invention is anhydrous calcium silicate.

In an especially preferred embodiment, the calcium silicate is calcium silicate hydrate. The calcium silicate hydrate for use in the present invention comprises at least calcium oxide (CaO), silica ($SiO_2$) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%.

The calcium silicate hydrate preferably comprises Ca and Si in an atom ratio (Ca:Si) less than 1:1, more preferably less than 1:1.2, more preferably still from 1:1.5 to 1:4 and most preferably from 1:1.7 to 1:3.

The calcium silicate is preferably particulate as this allows for maximum surface area for contact with dental tissue. Thus, preferably the composition comprises particles comprising the calcium silicate. More preferably the particles have a weight average particle size of five microns or less, and even more preferably from 10 to 100%, and especially, from 25 to 100%, and most especially, from 70% to 100% by weight of the particles comprising calcium silicate used in this invention have a particle size from 0.1 to 1.5 microns.

In addition to calcium oxide, silica and water, the particles which comprise the calcium silicate hydrate may comprise other components, such as metal cations, anions (such as phosphate) and the like. However, it is preferred that the particles comprise CaO, $SiO_2$ and water in an amount of at least 70% by weight of the particles, more preferably at least 80%, more preferably still at least 90% and even more preferably at least 95%. Most preferably the particles consist of (or at least consist essentially of) CaO, $SiO_2$ and water.

The calcium silicate haydrate for use in the present invention is commercially available around the world, for example, it is commercially available under the name of Sorbosil CA40 from PQ Corporation.

Typically, the oral care composition of the present invention comprises from 0.1 to 50% by weight of the calcium silicate, more preferably from 0.2 to 30%, most preferably from 1 to 20%, based on the total weight of the oral care composition and including all ranges subsumed therein.

HYALURONIC ACID AND/OR ITS DERIVATIVES

It is believed that hyaluronic acid and/or its derivatives, due to its strong adsorption to both tooth surface and actives, can facilitate the deposition of actives on the surface of teeth. In the present invention, hyaluronic acid and/or its derivatives can be used as a deposition aid in combination with silicate to enhance tooth remineralization efficacy on tooth surface.

Hyaluronic acid per se is of a formula $(C_{14}H_{21}NO_{11})_n$ and can be depicted as follows:

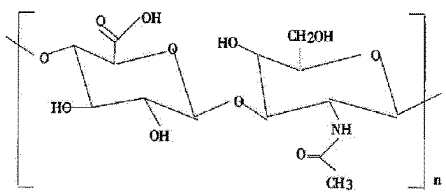

As can be learnt from the above formula, in one embodiment of the invention, the hyaluronic acid and/or its derivatives, refer to hyaluronic acid (an anionic, non-sulfated glycosaminoglycan), hyaluronate (like sodium hyaluronate, potassium hyaluronate, ammonium hyaluronate). In another embodiment of the invention, the hyaluronic acid and/or its derivatives refer to hyaluronic acid derivatives obtained by chemical modification (like HA-bisphosphonate, HA-amine/-HA-cystamine/HA-hydrazide/HA-dopamine/HA-thiol).

Specifically, the hyaluronic acid derivatives are hyaluronates in the form of a salt with a solubilizing cation, preferably sodium hyaluronate, potassium hyaluronate, ammonium hyaluronate or mixtures thereof. In another embodiment of the invention, the hyaluronic acid derivatives are modified hyaluronic acid with substituents of bisphosphonate,

amine, cystamine, hydrazide, dopamine, thiol or mixtures thereof.

The molecular weight for the hyaluronic acid and/or its derivatives ranges from 500,000 Dalton to 3,000,000 Dalton, preferably from 500,000 Dalton to 2,500,000 Dalton, more preferably still from 800,000 Dalton to 2,000,000 Dalton, most preferably from 1,000,000 Dalton to 2,000,000 Dalton.

In a particularly preferred embodiment of the invention, the hyaluronic acid and/or its derivatives suitable for use in the present application is sodium hyaluronate, which is commercially available from Bloomage Biotech, Shandong, China. Other suppliers for suitable hyaluronic acid and/or its derivatives comprise Shandong Focuschem Biotech Co., Ltd. (Focusbeauty HA®) and Topscience (Cosmehya®).

Typically, the oral care composition of the present invention comprises from 0.1 to 10% by weight, preferably from 0.2 to 5% by weight of hyaluronic acid and/or its derivatives, based on the total weight of the oral care composition and including all ranges subsumed therein.

Physiologically Acceptable Carrier

The composition of the present invention is an oral care composition and typically comprises a physiologically acceptable carrier. The physiologically acceptable carrier preferably comprises at least surfactant, thickener, humectant or a combination thereof.

Surfactant

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000

to 165,000 centipoise.

Humectant

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

Other ingredients

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitaminutes, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

Use and Manufacture

The oral care composition of this invention can be used in a method of remineralizing and/or reducing hypersensitivity of an individual comprising applying the composition to at least one surface of the teeth of the individual. The oral care composition of this invention may additionally or alternatively be for use as a medicament and/or used in the manufacture of a medicament for providing an oral care benefit as described herein, such as for remineralization of the teeth of an individual. Alternatively and preferably, the use is non-therapeutic.

Typically the composition will be packaged. In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention. The examples are not provided to limit the scope of the claims.

## Examples

### Example 1

This example listed dual-phase compositions for testing the blockage of dentinal tubules, and demonstrated the improved blockage efficacy of dentinal tubules by using calcium silicate in combination with hyaluronic acid and/or its derivatives as compared with control, the differences therebetween lie in only the amounts of addition of sodium hyaluronate. All ingredients are expressed by weight percentage of the total formulation, and as level of active ingredient.

**Protocol:** To evaluate blockage efficacy of dentinal tubules, serum slurries were made by mixing tube 1 and tube 2 in a ratio of 1:1 (w:w) firstly. Sample 1 was used as control.

Sample 1 (control sample)

**Table 1**

| Ingredients | |
|---|---|
| **Tube 1** | **Tube 2** |
| 53.94% glycerine, 10.50% PEG400, 2.00% PEG 3000, 1.11% sodium monofluorophosphate (SMFP), 0.25% sodium saccharin, 0.50% mica, 1.10% Flavour, 3.20% monosodium phosphate (MSP), 3.80% trisodium phosphate (TSP), 15.00% calcium silicate[a], 2.00% sodium lauryl sulfate (SLS), 6.00% silica abrasive (Zeodent 113), 0.5% silica abrasive (Sorbosil AC43), 0.1% thickener | 100% DI water |

a. Commercially available calcium silicate ($CaSiO_3$) Sorbosil CA40, from P.Q.Corporation

Sample 2

**Table 2**

| Ingredients | |
|---|---|
| **Tube 1** | **Tube 2** |
| 53.94% glycerine, 10.50% PEG400, 2.00% PEG 3000, 1.11% sodium monofluorophosphate (SMFP), 0.25% sodium saccharin, 0.50% mica, 1.10% Flavour, 3.20% monosodium phosphate (MSP), 3.80% trisodium phosphate (TSP), 15.00% calcium silicate[a] (CS), 2.00% sodium lauryl sulfate (SLS), 6.00% silica abrasive (Zeodent 113), 0.5% silica abrasive (Sorbosil AC43), 0.1% thickener | 0.5% sodium hyaluronate[b], 99.5% DI water |

b. Sodium hyaluronate ($M_v$: 1500 kDa) under the trade name Hybloom™ sodium hyaluronate (HA-T) commercially available from Bloomage Biotech, Shandong, China

Sample 3.

**Table 3**

| Ingredients | |
|---|---|
| **Tube 1** | **Tube 2** |
| 53.94% glycerine, 10.50% PEG400, 2.00% PEG 3000, 1.11% sodium monofluorophosphate (SMFP), 0.25% sodium saccharin, 0.50% mica, 1.10% Flavour, 3.20% monosodium phosphate (MSP), 3.80% trisodium phosphate (TSP), 15.00% calcium silicate[a] (CS), 2.00% sodium lauryl sulfate (SLS), 6.00% silica abrasive (Zeodent 113), 0.5% silica abrasive (Sorbosil AC43), 0.1% thickener | 1.0% sodium hyaluronate[b], 99.0% DI water |

Sample 4.

**Table 4**

| Ingredients | |
|---|---|
| **Tube 1** | **Tube 2** |
| 53.94% glycerine, 10.50% PEG400, 2.00% PEG 3000, 1.11% sodium monofluorophosphate (SMFP), 0.25% sodium saccharin, 0.50% mica, 1.10% Flavour, 3.20% monosodium phosphate (MSP), 3.80% trisodium phosphate (TSP), 15.00% calcium silicate[a] (CS), 2.00% sodium lauryl sulfate (SLS), 6.00% silica abrasive (Zeodent 113), 0.5% silica abrasive (Sorbosil AC43), 0.1% thickener | 1.5% sodium hyaluronate[b], 98.5% DI water |

Testing Methods

Human dentine discs were eroded by 6% citric acid for 3 min, then they were treated with different slurries via soaking following the same protocol. Eight human dentine discs were separated into four groups (n=2). Human dentine discs were soaked in the slurry for 15 minutes in a shaking water bath at 37°C and 60.0 rpm. Then, the human dentine discs were placed in 50 mL de-ionised (DI) water and agitated on a tooth brushing machine at 150 rpm for 1 min, so as to rinse the sample by distilled water (50 mL) and remove the excessive slurry on the surface of the human dentine discs. Afterwards, the samples were incubated in simulated oral fluid (SOF) under the condition of a shaking water bath at 37°C and 60.0 rpm for at least 6 h to mimic oral environment. Each dentine discs were treated twice using the protocol in the above testing method.

Simulated oral fluid (SOF) was made by combining the ingredients in Table 5.

**Table 5**

| Ingredient | Amount/g |
|---|---|
| NaCl | 16.07 |
| $NaHCO_3$ | 0.7 |
| KCl | 0.448 |
| $K_2HPO_4*3H_2O$ | 3.27 |
| $MgCl_2*6H_2O$ | 0.0622 |
| 1M HCl | 40 mL |
| $CaCl_2$ | 0.1998 |
| $Na_2SO_4$ | 0.1434 |
| Buffer | Adjust pH to 7.0 |
| Water | Balance to 2L |

Scoring Standard for Tubules Blockage

The treated dentine samples were characterized by scanning electron microscopy (SEM, Hitachi S-4800, Japan).

Regardless of the original shape of the dentine discs, a square (with a size of 4mm x 4mm) was selected and one image was captured under 50x magnification. Within this square, five spots (each with a size of 150 μm x 150 μm, one in the middle, and one in every corner) were selected and observed under 1000x magnification. The blockage of tubules was accessed following the standards described in Table 6. The measurement was carried out for the two dentine discs of each test group.

**Table 6**

| Score | Tubules Blockage |
|---|---|
| 0 | All dentinal tubules are open. |
| 1 | <20% of dentinal tubules are fully blocked. |
| 2 | 20 to 50% of dentinal tubules are fully blocked. |

(continued)

| Score | Tubules Blockage |
|---|---|
| 3 | 50 to 80% of dentinal tubules are fully blocked. |
| 4 | 80-100% of dentinal tubules are fully blocked. |
| 5 | All dentinal tubules are fully blocked. |

After treatment, SEM images of the dentine discs were taken. The images were analyzed and scored. The results for samples 1 to 4 were summarized in Table 7 (SE represents standard error for duplicate measurements).

Table 7

| | Sample 1 (control) | Sample 2 | Sample 3 | Sample 4 |
|---|---|---|---|---|
| Mean Score | 2.6 | 3.8 | 4.2 | 4.5 |
| SE | 0.5 | 0.3 | 0.2 | 0.2 |

The "p value" for the above samples was < 0.05.

SE represents standard error for duplicate measurements.
Samples 2 to 4 all showed significantly better (p<0.05) tubule blockage efficacy compared to sample 1.

Example 2

This example demonstrated the improved blockage of dentinal tubules by using sodium hyaluronate as a deposition aid of calcium silicate.

Table 8

| Ingredient | Sample 5 | Sample 6 | Sample 7 | Sample 8 | Sample 9 |
|---|---|---|---|---|---|
| Calcium silicate[a] | 0.75 g | 0.75g | 0.75g | / | / |
| Sodium hyaluronate ($M_v$: 1500 kDa)[b] | / | 0.05g | / | / | 0.05g |
| Sodium hyaluronate[c] ($M_v$: 400 kDa) | / | / | 0.05g | 0.05g | / |
| DI water | 10 mL | 10 mL | 10 mL | 10 mL | 10 mL |

c. Sodium hyaluronate commercially available under the trade name Hybloom™ HA-TLM 20-40 from Bloomage Biotech, Shandong, China

**Protocol: 3 brushings+ 1 min water brushing**

To evaluate blockage efficacy of dentinal tubules, ingredients of the samples were mixed to make a slurry. Sample 5 comprising only calcium silicate was used as control.
Human dentine discs were eroded by 6% citric acid for 3 minutes; then they were treated with different slurries via brushing following the same protocol. Ten human dentine discs were separated into five groups (n=2). The dentine discs were brushed with the slurry under a tooth brushing machine equipped with toothbrushes. The load of the tooth brushing was 170 g +/-5 g and the automatic brushing was operated at a speed of 150 rpm. After brushing for 1 min, the dentine discs were soaked in the toothpaste slurry for 1 min. Then the dentine discs were rinsed with distilled water and placed in simulated oral fluid (SOF) under the condition of a shaking water bath at 37 °C and 60.0 rpm. After soaking for about 3 to 4 hours, the dentine discs were brushed with the slurry by machine using the same procedure as in the first step. The brushing was repeated three times for one day, and the dentine discs were further kept in SOF overnight (>12 hours) in a shaking water bath at 37 °C to mimic oral environment. Then dentine discs were then brushed with DI water for 1 minute on the same tooth brushing machine. After brushings, SEM images of the dentine discs were taken. The images were analyzed and scored. The results were summarized in Table 9 below.

Table 9

| | Sample 5 | Sample 6 | Sample 7 | Sample 8 | Sample 9 |
|---|---|---|---|---|---|
| Mean Score | 2.7 | **4.3** | 3.0 | 2.2 | 1.2 |
| SE | 0.5 | 0.3 | 0.3 | 0.5 | 0.2 |

SE represents standard error for duplicate measurements.

Sample 6 comprising calcium silicate and sodium hyaluronate with a higher molecular weight showed significantly better ($p<0.05$) tubule blockage efficacy compared to other samples.

**Example 3.**

This example demonstrated the improved blockage of dentinal tubules by using sodium hyaluronate as a deposition aid of calcium silicate.

Table 10

| Ingredient | Sample 10 | Sample 11 |
|---|---|---|
| Calcium silicate[a] | 0.75 g | 0.75g |
| Sodium hyaluronate[b] (1500 kDa) | / | 0.05g |
| DI water | 10 mL | 10 mL |

**Protocol: 3 brushings only**

To evaluate blockage efficacy of dentinal tubules, ingredients of the samples were mixed to make a slurry. Sample 10 comprising only calcium silicate was used as control.

Human dentine discs were eroded by 6% citric acid for 3 minutes, then they were treated with different slurries via brushing following the same protocol. Four human dentine discs were separated into two groups (n=2). The dentine discs were brushed with the slurry under a tooth brushing machine equipped with toothbrushes. The load of the tooth brushing was 170 g +/-5 g and the automatic brushing operated at a speed of 150 rpm. After brushing for 1 min, the dentine discs were soaked in the toothpaste slurry for 1 min. Then the dentine discs were rinsed with distilled water and placed in simulated oral fluid (SOF) under the condition of a shaking water bath at 37 °C and 60.0 rpm. After soaking for about 3 to 4 hours, the dentine discs were brushed with the slurry by machine using the same procedure as in the first step. The brushing was repeated three times for one day, then the dentine discs were kept in SOF overnight (>12 hours) in a shaking water bath at 37 °C to mimic oral environment. Then dentine discs were observed by SEM. After brushings, SEM images of the dentine discs were taken. The images were analyzed and scored. The results were summarized in Table 11.

Table 11

| | Sample 10 | Sample 11 |
|---|---|---|
| Mean Score | 3.7 | **5** |
| SE | 0.5 | 0 |

The "p value" for the above samples was < 0.05.

SE represents standard error for duplicate measurements.

**Claims**

**1.** An oral care composition comprising:

a) calcium silicate; and
b) hyaluronic acid and/or its derivatives;

wherein the molecular weight of the hyaluronic acid and/or its derivatives ranges from 500,000 Dalton to 3,000,000 Dalton,
wherein the molecular weight refers to the viscosity average molecular weight and the viscosity average molecular weight of the hyaluronic acid and/or its derivatives is measured by using an Ubbelohde viscometer to obtain an intrinsic viscosity [η], and the molecular weight is calculated by the equation:

$$M_v = \left(\frac{[\eta]\times 10^5}{36}\right)^{\frac{1}{0.78}}$$

and wherein the hyaluronic acid derivatives are hyaluronates in the form of a salt with a solubilizing cation, preferably sodium hyaluronate, potassium hyaluronate, ammonium hyaluronate or mixtures thereof or modified hyaluronic acid with substituents of bisphosphonate, amine, cystamine, hydrazide, dopamine, thiol or mixtures thereof.

**2.** The oral care composition according to claim 1, wherein the hyaluronic acid derivatives are hyaluronates in the form of a salt with a solubilizing cation, preferably sodium hyaluronate, potassium hyaluronate, ammonium hyaluronate or mixtures thereof.

**3.** The oral care composition according to any of the preceding claims, wherein the hyaluronic acid and/or its derivatives are present in an amount of from 0.1 to 10% by weight of the composition, preferably from 0.2 to 5%.

**4.** The oral care composition according to any of the preceding claims, wherein the molecular weight of the hyaluronic acid and/or its derivatives ranges from 500,000 Dalton to 2,500,000 Dalton, preferably from 1,000,000 Dalton to 2,00,000 Dalton.

**5.** The oral care composition according to any of the preceding claims, wherein the calcium silicate is calcium silicate hydrate which comprises water of hydration in an amount of from 5 to 50% by weight of the calcium silicate hydrate, preferably from 10 to 40% by weight.

**6.** The oral care composition according to any of preceding claims 1 to 4 wherein the calcium silicate is not hydrated.

**7.** The oral care composition according to any of the preceding claims, wherein the calcium silicate is present in an amount from 0.1 to 50% by weight of the composition, preferably 1 to 20% by weight.

**8.** The oral care composition according to any of the preceding claims, further comprising physiologically acceptable carrier.

**9.** The oral care composition according to claim 8 wherein the physiologically acceptable carrier comprises at least surfactant, thickener, humectant or a combination thereof.

**10.** An oral care product, comprising the oral care composition according to any of the preceding claims.

**11.** An oral care composition for use in a method of remineralizing and/or reducing hypersensitivity of teeth of an individual, comprising the step of applying the oral care composition of any of the preceding claims to at least one surface of the teeth of the individual.

**12.** An oral care composition according to any one of the preceding claims 1 to 10 for use in tooth remineralization.

**Patentansprüche**

**1.** Mundpflegezusammensetzung, umfassend:

a) Calciumsilikat; und
b) Hyaluronsäure und/oder deren Derivate;

wobei das Molekulargewicht der Hyaluronsäure und/oder ihrer Derivate im Bereich von 500.000 Dalton bis 3.000.000 Dalton liegt,

wobei sich das Molekulargewicht auf das viskositätsmittlere Molekulargewicht bezieht und das viskositätsmittlere Molekulargewicht der Hyaluronsäure und/oder ihrer Derivate unter Verwendung eines Ubbelohde-Viskosimeters gemessen wird, um die intrinsische Viskosität [η] zu ermitteln, wobei das Molekulargewicht anhand der folgenden Gleichung berechnet wird:

$$M_v = \left(\frac{[\eta] \times 10^5}{36}\right)^{\frac{1}{0.78}},$$

und wobei die Hyaluronsäurederivate Hyaluronate in Form eines Salzes mit einem solubilisierenden Kation sind, vorzugsweise Natriumhyaluronat, Kaliumhyaluronat, Ammoniumhyaluronat oder Mischungen davon oder modifizierte Hyaluronsäure mit Substituenten aus Bisphosphonat, Amin, Cystamin, Hydrazid, Dopamin, Thiol oder Mischungen davon.

**2.** Mundpflegezusammensetzung nach Anspruch 1, wobei die Hyaluronsäurederivate Hyaluronate in Form eines Salzes mit einem solubilisierenden Kation sind, vorzugsweise Natriumhyaluronat, Kaliumhyaluronat, Ammoniumhyaluronat oder Mischungen davon.

**3.** Mundpflegezusammensetzung nach einem der vorhergehenden Ansprüche, wobei die Hyaluronsäure und/oder deren Derivate in einer Menge von 0,1 bis 10 Gew.-% der Zusammensetzung vorliegen, vorzugsweise von 0,2 bis 5 Gew.-%.

**4.** Mundpflegezusammensetzung nach einem der vorhergehenden Ansprüche, wobei das Molekulargewicht der Hyaluronsäure und/oder ihrer Derivate im Bereich von 500.000 Dalton bis 2.500.000 Dalton liegt, vorzugsweise von 1.000.000 Dalton bis 2.000.000 Dalton.

**5.** Mundpflegezusammensetzung nach einem der vorhergehenden Ansprüche, wobei das Calciumsilikat Calciumsilikathydrat ist, das Hydratwasser in einer Menge von 5 bis 50 Gew.-% des Calciumsilikathydrats umfasst, vorzugsweise von 10 bis 40 Gew.-%.

**6.** Mundpflegezusammensetzung nach einem der vorhergehenden Ansprüche 1 bis 4, wobei das Calciumsilikat nicht hydratisiert ist.

**7.** Mundpflegezusammensetzung nach einem der vorhergehenden Ansprüche, wobei das Calciumsilikat in einer Menge von 0,1 bis 50 Gew.-% der Zusammensetzung vorliegt, vorzugsweise von 1 bis 20 Gew.-%.

**8.** Mundpflegezusammensetzung nach einem der vorhergehenden Ansprüche, die ferner einen physiologisch verträglichen Träger umfasst.

**9.** Mundpflegezusammensetzung nach Anspruch 8, wobei der physiologisch verträgliche Träger mindestens ein Tensid, ein Verdickungsmittel, ein Feuchthaltemittel oder ein Kombination davon umfasst.

**10.** Mundpflegeprodukt, das die Mundpflegezusammensetzung nach einem der vorhergehenden Ansprüche umfasst.

**11.** Mundpflegezusammensetzung zur Verwendung in einem Verfahren zur Remineralisierung und/oder Verringerung der Überempfindlichkeit der Zähne einer Person, umfassend den Schritt des Auftragens der Mundpflegezusammensetzung nach einem der vorhergehenden Ansprüche auf mindestens eine Oberfläche der Zähne der Person.

**12.** Mundpflegezusammensetzung nach einem der vorhergehenden Ansprüche 1 bis 10 zur Verwendung bei der Remineralisierung der Zähne.

## Revendications

**1.** Composition d'hygiène buccale comprenant :

a) du silicate de calcium ; et
b) de l'acide hyaluronique et/ou ses dérivés ;

dans laquelle le poids moléculaire de l'acide hyaluronique et/ou de ses dérivés est situé dans la plage allant de 500 000 Daltons à 3 000 000 Daltons,
dans laquelle le poids moléculaire se réfère au poids moléculaire moyen en viscosité et le poids moléculaire moyen en viscosité de l'acide hyaluronique et/ou de ses dérivés est mesuré par utilisation d'un viscosimètre d'Ubbelohde pour que soit obtenue la viscosité intrinsèque [η], et le poids moléculaire est calculé par l'équation :

$$M_v = \left(\frac{[\eta]\times 10^5}{36}\right)^{\frac{1}{0.78}}$$

et dans laquelle les dérivés d'acide hyaluronique sont des hyaluronates sous la forme d'un sel avec un cation solubilisant, de préférence le hyaluronate de sodium, le hyaluronate de potassium, le hyaluronate d'ammonium ou leurs mélanges, ou de l'acide hyaluronique modifié avec des substituants bisphosphonate, amine, cystamine, hydrazide, dopamine, thiol ou leurs mélanges.

**2.** Composition d'hygiène buccale selon la revendication 1, dans laquelle les dérivés d'acide hyaluronique sont des hyaluronates sous la forme d'un sel avec un cation solubilisant, de préférence le hyaluronate de sodium, le hyaluronate de potassium, le hyaluronate d'ammonium ou leurs mélanges.

**3.** Composition d'hygiène buccale selon l'une quelconque des revendications précédentes, dans laquelle l'acide hyaluronique et/ou ses dérivés sont présents en une quantité de 0,1 à 10 %, de préférence de 0,2 à 5 %, en poids de la composition.

**4.** Composition d'hygiène buccale selon l'une quelconque des revendications précédentes, dans laquelle le poids moléculaire de l'acide hyaluronique et/ou de ses dérivés est situé dans la plage allant de 500 000 Daltons à 2 500 000 Daltons, de préférence de 1 000 000 Daltons à 2 000 000 Daltons.

**5.** Composition d'hygiène buccale selon l'une quelconque des revendications précédentes, dans laquelle le silicate de calcium est du silicate de calcium hydraté qui comprend de l'eau d'hydratation en une quantité de 5 à 50 % en poids, de préférence de 10 à 40 % en poids du silicate de calcium hydraté.

**6.** Composition d'hygiène buccale selon l'une quelconque des revendications 1 à 4, dans laquelle le silicate de calcium n'est pas hydraté.

**7.** Composition d'hygiène buccale selon l'une quelconque des revendications précédentes, dans laquelle le silicate de calcium est présent en une quantité de 0,1 à 50 % en poids, de préférence de 1 à 20 % en poids, de la composition.

**8.** Composition d'hygiène buccale selon l'une quelconque des revendications précédentes, comprenant en outre un véhicule physiologiquement acceptable.

**9.** Composition d'hygiène buccale selon la revendication 8, dans laquelle le véhicule physiologiquement acceptable comprend au moins un tensioactif, un épaississant, un humectant ou une combinaison de ceux-ci.

**10.** Produit d'hygiène buccale comprenant la composition d'hygiène buccale selon l'une quelconque des revendications précédentes.

**11.** Composition d'hygiène buccale pour une utilisation dans une méthode de reminéralisation et/ou de réduction de l'hypersensibilité des dents d'un individu, comprenant l'étape d'application de la composition d'hygiène buccale de l'une quelconque des revendications précédentes sur au moins une surface des dents de l'individu.

**12.** Composition d'hygiène buccale selon l'une quelconque des revendications 1 à 10, pour une utilisation dans la reminéralisation des dents.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017198392 A
- US 2019262254 A
- US 2013164359 A
- WO 2008068248 A, Unilever

### Non-patent literature cited in the description

- *CHEMICAL ABSTRACTS*, 9063-87-0
- *CHEMICAL ABSTRACTS*, 9004-32-4